(19) **Europäisches Patentamt European Patent Office Office européen des brevets**

(11) **EP 4 382 887 B1**

(12) **FASCICULE DE BREVET EUROPEEN**

(45) Date de publication et mention de la délivrance du brevet:
**14.01.2026 Bulletin 2026/03**

(21) Numéro de dépôt: **23209961.4**

(22) Date de dépôt: **15.11.2023**

(51) Classification Internationale des Brevets (IPC):
**G01N 15/10** (2024.01)  **B01L 3/00** (2006.01)
**G01N 29/036** (2006.01)  **G01N 33/543** (2006.01)
**G01N 5/02** (2006.01)  **G01G 3/16** (2006.01)
**G01H 13/00** (2006.01)

(52) Classification Coopérative des Brevets (CPC):
**G01N 15/10; B01L 3/502761; G01G 3/16; G01H 13/00; G01N 5/02; G01N 29/036; G01N 33/54313; G01N 33/54373;** B01L 2200/0668; B01L 2300/0883; B01L 2400/0433; G01N 2015/1006; G01N 2015/1021; G01N 2291/02466

(54) **PROCÉDÉ DE DÉTECTION DE CIBLES À L'AIDE D'UN OSCILLATEUR MÉCANIQUE ET DE PARTICULES FONCTIONNALISÉES**

VERFAHREN ZUR ZIELERKENNUNG MIT EINEM MECHANISCHEN OSZILLATOR UND FUNKTIONALISIERTEN PARTIKELN

METHOD FOR DETECTING TARGETS USING A MECHANICAL OSCILLATOR AND FUNCTIONALIZED PARTICLES

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **07.12.2022 FR 2212917**

(43) Date de publication de la demande:
**12.06.2024 Bulletin 2024/24**

(73) Titulaire: **Commissariat à l'Energie Atomique et aux Energies Alternatives**
**75015 Paris (FR)**

(72) Inventeurs:
• **AGACHE, Vincent**
  **38054 Grenoble cedex 09 (FR)**
• **CAILLAT, Patrice**
  **38054 Grenoble cedex 09 (FR)**

(74) Mandataire: **INNOV-GROUP**
**209 Avenue Berthelot**
**69007 Lyon (FR)**

(56) Documents cités:
**EP-A1- 2 574 885**

• **LEE JUNGCHUL ET AL: "High precision particle mass sensing using microchannel resonators in the second vibration mode", vol. 82, no. 2, 17 February 2011 (2011-02-17), pages 23704 - 23704, XP012146161, ISSN: 0034-6748, Retrieved from the Internet <URL:https://www.ncbi.nlm.nih.gov/pmc/articles/PMC3055905/> [retrieved on 20230620], DOI: 10.1063/1.3534825**
• **OLCUM SELIM ET AL: "High-speed multiple-mode mass-sensing resolves dynamic nanoscale mass distributions", vol. 6, no. 1, 12 May 2015 (2015-05-12), XP093001718, Retrieved from the Internet <URL:http://www.nature.com/articles/ncomms8070> [retrieved on 20230621], DOI: 10.1038/ncomms8070**

EP 4 382 887 B1

## Description

### Domaine technique de l'invention

La présente invention se rapporte à un procédé de détection de cibles (ou biomarqueurs) à l'aide d'un oscillateur mécanique et de particules fonctionnalisées.

### Etat de la technique

Pour détecter la présence de biomarqueurs d'intérêt tels que des protéines, exosomes, ARN circulant, ADN circulant, virus, ou des espèces cibles de plus grandes dimensions telles que bactéries et cellules, il est connu d'utiliser des biocapteurs. Dans les biocapteurs, il existe notamment les capteurs gravimétriques. Ceux-ci sont basés sur l'utilisation d'un oscillateur ou résonateur mécanique, mis en vibration sur sa fréquence de résonance. Toute cible qui se greffe sur l'oscillateur, entraîne l'augmentation de la masse de celui-ci, ce qui diminue sa fréquence de résonance, d'un décalage proportionnel à la masse de la cible.

En mesurant les fluctuations de fréquence de résonance en continu, il est alors possible de remonter à la masse adsorbée sur l'oscillateur en temps réel et suivre ainsi des cinétiques d'adsorption avec des cibles. La plupart des approches sont cependant basées sur une immersion de l'oscillateur dans le fluide à analyser. Un inconvénient majeur est alors l'amortissement de l'oscillation du résonateur par le fluide (visqueux), dégradant son facteur de qualité mécanique (Q-10 à 100 typiquement lorsque le résonateur est immergé) et par conséquent la limite de détection du capteur. De plus, pour conférer à la mesure une spécificité, une fonctionnalisation préalable du capteur est requise, ce qui peut compliquer son intégration dans un canal microfluidique en raison des contraintes de localisation des sondes, et de compatibilité entre la méthode employée pour immobilisation des sondes avec les matériaux en présence. Pour remédier à l'amortissement du fluide, une alternative initialement proposée par l'équipe du professeur Scott Manalis au "MIT" consiste à intégrer et délimiter un canal fluidique à l'intérieur même de l'oscillateur, tandis que celui-ci oscille dans une cavité exempte de fluide. L'avantage réside alors en un facteur de qualité très peu altéré et une limite de détection optimisée, même en présence de fluide circulant dans l'oscillateur. Ce type d'oscillateur est couramment appelé SMR ("Suspended Microchannel Resonator") ou encore SNR ("Suspended Nanochannel Resonator"), selon les dimensions du canal. Par un gradient de pression imposé entre une entrée fluidique et une sortie fluidique du circuit, il est possible de contrôler l'écoulement du fluide (son débit et sens d'écoulement), et donc le passage de particules en suspension dans le SMR (ou SNR). Ce type de capteur a été utilisé pour diverses applications, incluant la pesée individuelle de particules biologiques telles que des cellules, bactéries, des nanoparticules ou encore la détection de protéines spécifiques par la fonctionnalisation préalable des parois internes du SMR. Ce principe de fonctionnement est désormais bien connu.

La publication référencée "Y.C. Weng, F.F. Delgado, S. Son, T.P. Burg, S.C. Wasserman, S.R. Manalis. Mass sensors with mechanical traps for weighing single cells in different fluids, Lab on a chip (2011*)"* décrit un oscillateur muni d'un système de piégeage d'une particule biologique. L'oscillateur peut notamment comporter une poutre munie d'un canal d'aspiration central, avec une restriction à son extrémité libre, formant un piège pour une particule.

Il est également connu de la demande de brevet EP2574885A1 un dispositif de détection massique de particules. Ce dispositif comporte un circuit fluidique intégré dans un oscillateur électromécanique. Le circuit fluidique comporte un ou plusieurs pièges pour des particules injectées dans le circuit fluidique. Les particules peuvent notamment être des billes dont la surface est fonctionnalisée de manière à former une surface de capture d'une espèce cible présente dans un liquide injecté dans le circuit fluidique. Au fur et à mesure que la capture des espèces cibles s'effectue, la masse de l'oscillateur s'accroît, entraînant une variation de la fréquence de résonance, cette variation permettant ensuite de déduire la masse de l'espèce cible collectée sur chaque bille.

Les solutions antérieures ne permettent cependant pas d'exploiter au maximum les capacités de l'oscillateur, ni de détecter la présence de plusieurs cibles à l'aide d'un même oscillateur.

Un premier but de l'invention est ainsi de proposer une solution qui permet d'exploiter au mieux les capacités d'un oscillateur tel que ceux déjà décrits ci-dessus.

Un deuxième but de l'invention est de proposer une solution pour permettre la détection de plusieurs cibles contenues dans un même liquide et à l'aide d'un seul oscillateur.

### Exposé de l'invention

Ce but est atteint par un procédé de détection d'une première cible et d'une deuxième cible, mis en œuvre à l'aide d'un système de détection qui comporte :

- Un dispositif de mesure qui comporte un oscillateur mécanique et un circuit fluidique intégré à l'oscillateur et dans lequel est amené à circuler un fluide contenant ladite première cible et ladite deuxième cible à détecter, ledit oscillateur étant apte à être excité selon plusieurs modes de vibration et comportant sur le réseau fluidique au moins un premier piège positionné sur un anti-nœud d'un premier mode de vibration de l'oscillateur et un deuxième piège positionné sur un anti-nœud d'un deuxième mode de vibration de l'oscillateur, un anti-nœud de vibration correspondant à une position dans laquelle l'amplitude de vibration va de 80% à 100% de l'amplitude maximale pour le mode de vibration sélectionné,

- Des moyens d'injection d'un fluide dans le circuit fluidique,
- Des moyens d'excitation dudit oscillateur selon le premier mode de vibration et/ou le deuxième mode de vibration,
- Des moyens de mesure de la fréquence de résonance de l'oscillateur, Ledit procédé comportant des étapes de :
- Piégeage d'une première particule dans le premier piège de l'oscillateur et piégeage d'une deuxième particule dans le deuxième piège de l'oscillateur, ladite première particule disposant d'une surface externe fonctionnalisée à l'aide d'une première sonde complémentaire de ladite première cible et ladite deuxième particule disposant d'une surface externe fonctionnalisée à l'aide d'une deuxième sonde complémentaire de ladite deuxième cible,
- Injection du fluide dans le circuit fluidique, ledit fluide contenant ladite première cible et ladite deuxième cible à détecter,
- Première excitation de l'oscillateur selon le premier mode de vibration et mesure d'une première fréquence de résonance de l'oscillateur,
- Détection de ladite première cible à partir d'une variation de ladite première fréquence de résonance,
- Deuxième excitation de l'oscillateur selon le deuxième mode de vibration et mesure d'une deuxième fréquence de résonance de l'oscillateur,
- Détection de ladite deuxième cible à partir d'une variation de ladite deuxième fréquence de résonance.

Selon une réalisation particulière, la première sonde et la deuxième sonde sont distinctes pour capturer respectivement la première cible et la deuxième cible qui sont distinctes. Selon une autre réalisation particulière, la première sonde et la deuxième sonde sont identiques pour capturer la première cible et la deuxième cible qui sont identiques.

Selon une particularité, le procédé comporte une étape d'injection dans le circuit fluidique de particules dites secondaires fonctionnalisées chacune par une sonde spécifique de la première cible et/ou de la deuxième cible.

Selon une autre particularité, chaque particule est réalisée sous la forme d'une bille rigide.

Selon une autre particularité, les billes sont de tailles différentes.

Selon une autre particularité, la première excitation et la deuxième excitation sont réalisées de manière simultanée.

L'invention concerne également un système de détection selon la revendication 8 employé pour mettre en œuvre le procédé de caractérisation de particules, ledit système comprenant :

- Un dispositif de mesure qui comporte un oscillateur mécanique et un circuit fluidique intégré à l'oscillateur et dans lequel est amené à circuler un fluide contenant les particules à caractériser, ledit oscillateur étant apte à être excité selon plusieurs modes de vibration et comportant sur le réseau fluidique au moins un premier piège positionné sur un anti-nœud d'un premier mode de vibration de l'oscillateur et un deuxième piège positionné sur un anti-nœud d'un deuxième mode de vibration de l'oscillateur, un anti-nœud de vibration correspondant à une position dans laquelle l'amplitude de vibration va de 80% à 100% de l'amplitude maximale pour le mode de vibration sélectionné,
- Des moyens d'injection d'un fluide dans le circuit fluidique,
- Des moyens d'excitation dudit oscillateur selon le premier mode de vibration et/ou le deuxième mode de vibration,
- Des moyens de mesure de la fréquence de résonance de l'oscillateur.

Selon une particularité, le système comporte des moyens de détection de la variation de la fréquence de résonance.

Selon une autre particularité, l'oscillateur comporte une poutre fixée en porte à faux et qui intègre le circuit fluidique.

Selon une réalisation particulière, le circuit fluidique comporte un canal principal et un canal intermédiaire, chaque piège fluidique étant réalisé sous la forme d'une cavité entre le canal principal et le canal intermédiaire, qui comporte une partie élargie prolongée d'une restriction.

Selon une autre réalisation particulière, le circuit fluidique comporte un canal principal s'étendant entre une entrée fluidique et une sortie fluidique, le canal principal comportant une première branche dans laquelle débouche l'entrée fluidique, une deuxième branche prolongeant ladite première branche, via une branche de jonction, et débouchant sur la sortie fluidique, chaque piège fluidique étant réalisé sous la forme d'une branche de dérivation par rapport au canal principal et comportant un logement dimensionné pour accueillir une particule et une restriction prolongeant ledit logement.

## Brève description des figures

D'autres caractéristiques et avantages vont apparaître dans la description détaillée qui suit, faite en liaison avec les figures listées ci-dessous :

- Les figures 1A et 1B représentent le système de détection de l'invention, respectivement selon deux variantes de réalisation de son dispositif de mesure ;

- La figure 2 montre des exemples de courbes de déformée de l'oscillateur du dispositif de la figure 1A et de la figure 1B selon différents modes de vibration ;
- La figure 3 montre une première configuration de fonctionnement du système de l'invention ;
- La figure 4 montre une deuxième configuration de fonctionnement du système de l'invention ;
- La figure 5 illustre une autre configuration de fonctionnement du système de l'invention ;
- La figure 6 représente un tableau reprenant les performances attendues pour différents oscillateurs ;

**Description détaillée d'au moins un mode de réalisation**

L'invention vise à détecter plusieurs cibles de manière simultanée en utilisant des particules fonctionnalisées, en utilisant un même oscillateur.

Les particules sont préférentiellement des entités suffisamment rigides. On utilise avantageusement des billes dont la surface externe est fonctionnalisée par une ou plusieurs sondes choisies pour capturer des cibles présentes dans un liquide L. De manière non limitative, dans la suite de la description, on pourra parler de billes ou de particules fonctionnalisées.

L'invention vise notamment la détection de cibles telles que protéines, exosomes, ARN circulant, ADN circulant, virus, présents dans le liquide injecté dans le circuit fluidique et captés par la surface fonctionnalisée des particules.

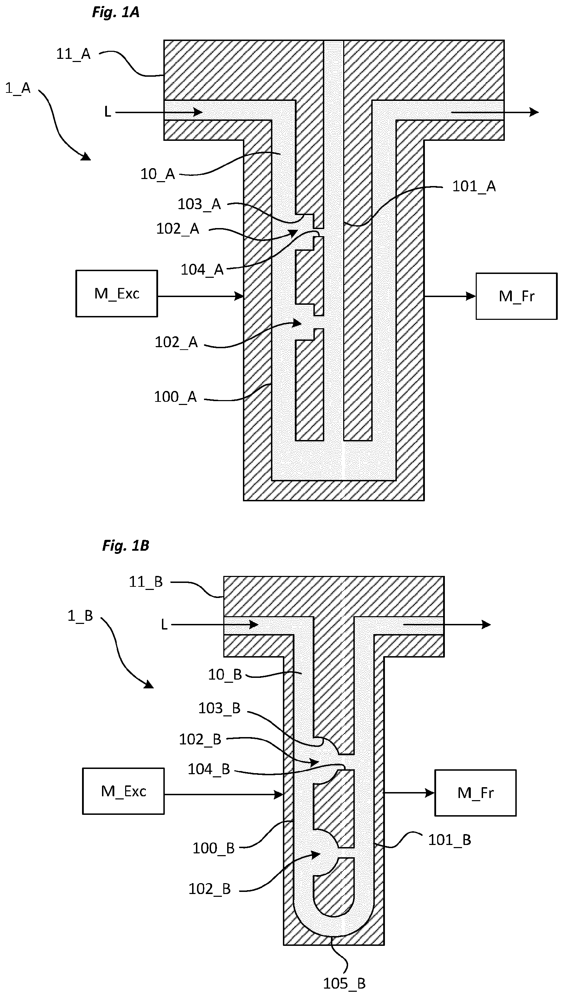

Les figures 1A et 1B montrent le système de détection pouvant être utilisé dans la mise en œuvre du procédé de caractérisation de l'invention.

Le système de détection comporte un dispositif de mesure 1_A, 1_B mécanique et fluidique, des moyens d'excitation M_Exc et des moyens de mesure M_Fr.

Le dispositif de mesure 1 comporte un circuit fluidique 10_A, 10_B dans lequel vont être injectées les particules.

Le dispositif de mesure 1_A, 1_B comporte également un oscillateur 11_A, 11_B, connu également sous le nom de résonateur, de type SMR ou SNR (désigné ci-après "oscillateur") le circuit fluidique 10_A, 10_B étant intégré à l'oscillateur.

L'oscillateur 11_A, 11_B comporte une poutre fixée en porte-à-faux de manière à présenter une extrémité libre. La poutre est agencée pour osciller à l'intérieur d'une cavité, préférentiellement une cavité sous vide afin de réduire l'amortissement de la poutre en vibration par les molécules d'air. La poutre est capable d'être excitée selon plusieurs modes de vibration, chacun de ces modes se traduisant par une déformation de la poutre présentant localement des nœuds et ventres de vibration (ou ci-après anti-nœuds) à différents endroits prédéterminés par les lois de la mécanique et dynamique des structures.

Le circuit fluidique 10_A, 10_B est intégré à la poutre de l'oscillateur.

Le principe de base repose sur le piégeage de plusieurs particules fonctionnalisées à l'intérieur de l'oscillateur 11_A, 11_B.

Selon une première variante de réalisation représentée sur la figure 1A, le circuit fluidique 10_A comporte un canal principal 100_A et un canal intermédiaire 101_A. Le circuit fluidique 10_A est arrangé de manière à présenter plusieurs pièges fluidiques 102_A, destinés chacun à pouvoir piéger une particule distincte.

Chaque piège fluidique 102_A est réalisé sous la forme d'une intersection entre le canal principal 100_A et le canal intermédiaire 101_A. Un piège fluidique comporte une cavité débouchant d'un côté sur le canal principal 100_A et de l'autre côté sur le canal intermédiaire 101_A. Du côté du canal principal, la cavité comporte une partie élargie 103_A pour accueillir la particule et, du côté du canal intermédiaire, elle comporte une restriction 104_A ou un étranglement.

On applique un gradient de pression entre le canal principal 100_A et le canal intermédiaire 101_A, de sorte qu'une fraction du flux principal est aspirée dans le canal intermédiaire, via chaque piège fluidique.

Le gradient de pression permet de capturer (par aspiration) toute particule suffisamment rigide (ou entité biologique plus ou moins rigide également) circulant dans le canal principal 100_A et dont le diamètre est supérieur à la restriction créée dans le piège fluidique considéré. La rigidité de la particule l'empêche de traverser la restriction. Par extension de ce principe, en disposant de N pièges fluidiques, il est possible de capturer N particules. Les particules ou billes fonctionnalisées vont ainsi pouvoir être piégées dans chaque piège fluidique.

La taille de la partie élargie de la cavité doit être adaptée à celle de la particule à accueillir pour que cette dernière puisse rester piégée.

Selon une autre variante de réalisation représentée sur la figure 1B, le circuit fluidique 10_B du dispositif de mesure 1_B comporte un canal principal s'étendant entre une entrée fluidique et une sortie fluidique. Le canal principal comporte une première branche 100_B dans laquelle débouche l'entrée fluidique, une deuxième branche 101_B prolongeant ladite première branche, via une première branche de jonction 105_B, et débouchant sur la sortie fluidique. Le circuit fluidique 10_B comporte plusieurs pièges fluidiques. Chaque piège fluidique 102_B est réalisé sous la forme d'une branche de dérivation par rapport au canal principal et comporte un logement 103_B dimensionné pour accueillir une particule et une restriction 104_B prolongeant ledit logement. Chaque piège fluidique est agencé de sorte que son logement 103_B communique d'un côté avec la première branche 100_B du canal principal et que sa restriction 104_B débouche de l'autre côté dans la deuxième branche 101_B du canal principal. A l'aide de ce circuit fluidique 10_B, le piégeage de particules se fait sans élément de contrôle externe, par un système hydrodynamique. L'avantage d'un piège hydrodynamique réside dans le fait de ne pas nécessiter la présence d'un canal intermédiaire et donc de source de pression supplémentaire pour aspirer la ou les particules. Le logement et la restriction sont conçus de sorte que la résistance hydraulique de la restriction soit inférieure à la résistance hydraulique au niveau du canal principal. Ainsi la particule se loge préférentiellement dans le premier logement libre qui se présente sur son trajet, tandis que les particules suivantes poursuivent leur trajectoire.

Ci-après, on conserve des références générales pour les éléments communs aux deux variantes de réalisation du dispositif de mesure.

Partant des deux arrangements possibles du dispositif de mesure 1, on place ainsi plusieurs pièges fluidiques 102 sur l'oscillateur 11 le long du circuit fluidique 10.

Selon un aspect particulier de l'invention, chaque piège fluidique 102 est positionné sur un antinœud ou ventre de vibration différent, associé chacun à un mode de vibration distinct de l'oscillateur.

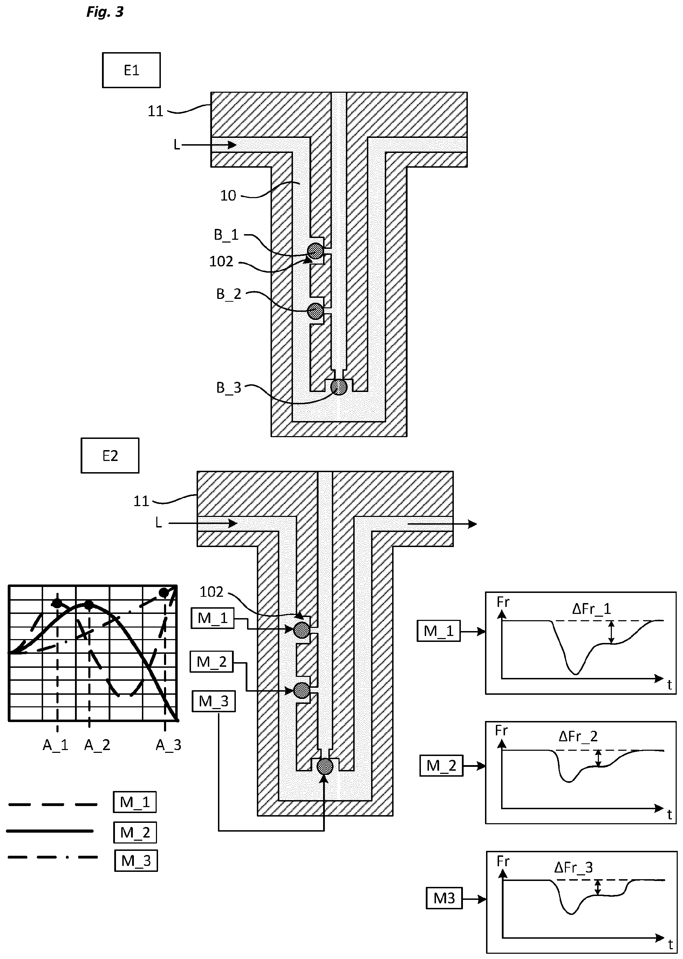

Autrement dit, avec un oscillateur 11 qui comporte par exemple trois pièges fluidiques (tel que représenté sur la figure 3), on a :

- Le premier piège fluidique placé sur un anti-nœud A_1 d'un premier mode de vibration M_1 de son oscillateur 11 ;
- Le deuxième piège fluidique placé sur un anti-nœud A_2 d'un deuxième mode de vibration M_2 de son oscillateur 11 ;
- Le troisième piège fluidique placé sur un anti-nœud A_3 d'un troisième mode de vibration M_3 de son oscillateur 11 ;

Pour rappel, un anti-nœud de vibration correspond à une zone de piégeage où l'amplitude de vibration est maximale. Dans le cadre de l'invention, on pourra considérer que le piège fluidique est bien sur un antinœud de vibration lorsque l'amplitude de vibration va de 80% à 100% de l'amplitude maximale pour le mode de vibration sélectionné.

Avantageusement, le piège fluidique sera positionné de manière à correspondre à une amplitude de vibration égale à 100% de de l'amplitude de vibration maximale pour le mode de vibration sélectionné.

L'emplacement d'un piège fluidique 102 au niveau d'un anti-nœud de vibration est avantageux car il augmente la sensibilité de détection de l'oscillateur pour le mode de vibration correspondant par rapport à la particule piégée dans ledit emplacement.

En effet, lorsqu'une particule de masse $\Delta m$ est piégée dans un piège fluidique de l'oscillateur, le décalage de la fréquence de résonance $\Delta f$ de l'oscillateur dépend de la masse ajoutée $\Delta m$, de la masse totale m de l'oscillateur ainsi que d'un coefficient de correction $\alpha$ qui dépend spécifiquement de la position de la particule ajoutée, selon la relation suivante :

$$\frac{\Delta f}{f} = -\propto \frac{\Delta m}{m}$$

Ainsi, en piégeant une particule de masse ponctuelle $\Delta m$ donnée dans une position présentant un maximum d'amplitude de vibration, on obtient un décalage de fréquence maximum. Autrement dit, le piégeage de la particule dans une telle position (au niveau d'un antinœud) permet de maximiser le coefficient de correction $\alpha$ et par conséquent, d'accroître la sensibilité du dispositif.

Le système comporte en outre des moyens de mise en vibration ou moyens d'excitation de l'oscillateur. Il peut s'agir d'une excitation réalisée par un mode capacitif, piézoélectrique, électromagnétique, thermique, thermo-élastique et optique.

Les moyens de mise en vibration M_Exc sont commandés de manière à pouvoir exciter l'oscillateur 11 selon plusieurs modes de vibration distincts.

Selon un aspect particulièrement avantageux, on peut tirer profit de la mesure piézorésistive intégrée à l'oscillateur pour faire une détection multimodale. En l'occurrence, il s'agira de mesurer simultanément plusieurs modes de résonance de l'oscillateur, entrainés par l'actionnement de la piézocéramique située en dessous du composant. Plus particulièrement, la configuration des jauges piézorésistives et de la céramique permettront d'exciter et lire simultanément les fréquences de résonance associées à chaque mode de flexion hors plan de la poutre de l'oscillateur, même si d'autres modes tels que les modes dans le plan ou en torsion par exemple pourront être sollicités et mesurés.

Il serait également possible d'exciter l'oscillateur 11 selon chaque mode de vibration, de manière distincte et successive (non simultanée), pour interroger chaque piège fluidique de manière indépendante.

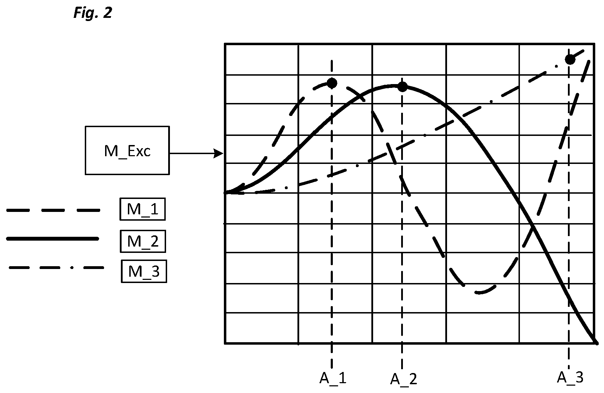

La figure 2 montre un diagramme représentant plusieurs courbes d'excitation de l'oscillateur, chaque courbe correspondant à la déformée résultant d'un mode de vibration M_1, M_2, M_3 distinct de l'oscillateur. Cette figure 2 indique également la position d'un anti-nœud A_1, A_2, A_3 associé à chacun des trois modes de vibrations.

Le système comporte également des moyens de mesure M_Fr de la fréquence de résonance de l'oscillateur 11. Il peut s'agir de moyens matériels et logiciels permettant de mesurer la fréquence de résonance (notamment par lecture du courant piézorésistif circulant dans la jauge piézorésistive implantée dans l'oscillateur 11, et dont les fluctuations de fréquence sont directement reliées aux fluctuations de fréquence de résonance de l'oscillateur sur chacun de ses modes de vibrations sollicités) et de moyens de détection des variations de cette fréquence (notamment un circuit électronique d'amplification du courant piézorésistif par pont de Wheatstone, suivi d'une électronique numérique embarquée dans une carte de type FPGA qui va permettre de lire numériquement ces signaux, les traduire en mesures de fluctuation de fréquence de résonance et permettre un asservissement avec un actionnement piézoélectrique). Dans le cadre de l'invention, plusieurs billes sont par exemple injectées successivement dans le circuit fluidique 10. Chaque bille est amenée à être piégée sur un site correspondant à un anti-nœud d'un mode de vibration distinct de l'oscillateur. Chaque bille dispose d'une surface fonctionnalisée de manière à pouvoir capturer une cible. De manière non limitative, on peut distinguer deux configurations de fonctionnement distinctes :

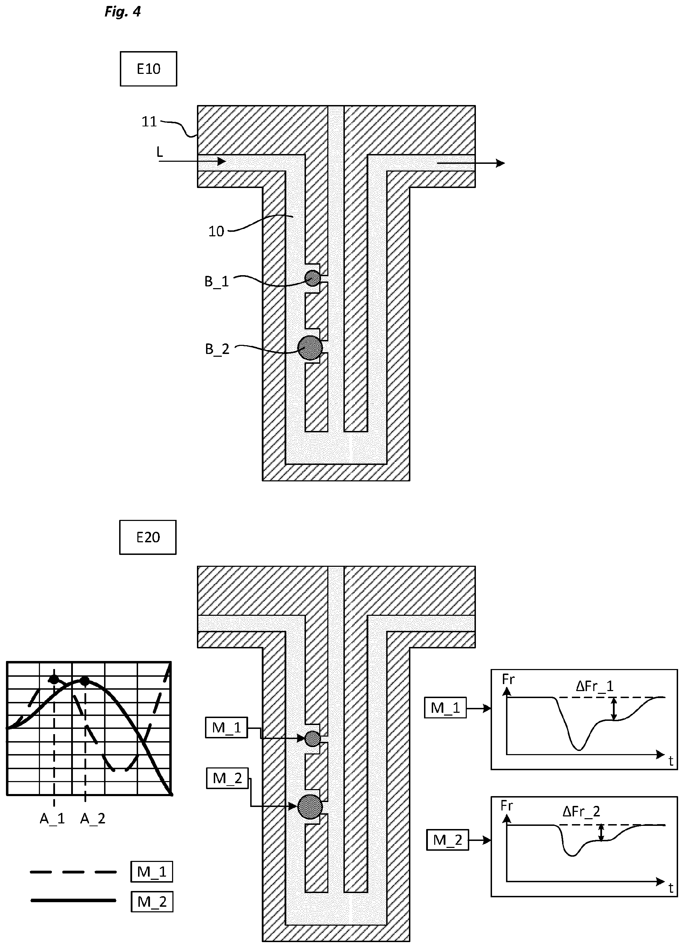

- Dans une première configuration illustrée par la figure 3, on utilise un oscillateur 11 disposant de pièges fluidiques 102 tous identiques, destinés à piéger des billes toutes identiques. On peut ainsi mesurer l'adsorption d'un seul type de cible sur une plus grande surface développée conférée par l'ensemble des billes, en isolant chaque bille sur un anti-nœud distinct pour avoir un maximum de sensibilité de détection.
- Dans une deuxième configuration illustrée par la figure 4, on utilise un oscillateur 11 disposant de pièges fluidiques 102 ayant des dimensions distinctes au niveau de leur logement d'accueil et de leur restriction, de manière à pouvoir accueillir des billes de tailles distinctes. On peut ainsi procéder à une analyse multi-cibles.

En liaison avec la figure 3, l'oscillateur 11 est donc muni de pièges fluidiques 102 tous identiques et de billes toutes identiques, les billes ayant chacune une surface fonctionnalisée de manière identique pour capturer une cible unique présente dans le liquide L injecté dans le circuit fluidique 10.

Dans cette réalisation, le fait de piéger plusieurs billes permet d'accroître la surface développée et donc la quantité de sondes accessibles pour le greffage des cibles, tout en exploitant les trois modes de vibrations et les trois anti-nœuds correspondants, pour également accroître la sensibilité de détection en comparaison d'une fonctionnalisation intégrale des parois du capteur.

Selon la première configuration, le processus de détection est décrit ci-dessous en liaison avec la figure 3 :

E1 : Injection de plusieurs billes B_1, B_2, B_3, par exemple au nombre de trois. Chaque bille vient se placer dans un piège fluidique 102 distinct.

Chaque bille se charge de la cible présente dans le liquide L injecté dans le circuit fluidique 10.

E2 : Le système permet ensuite de venir interroger les sites de piégeage de manière simultanée en appliquant les différents modes de vibration M_1, M_2, M_3 de l'oscillateur ou de manière indépendante en sélectionnant un mode de vibration parmi les différents modes de vibration M_1, M_2, M_3 de l'oscillateur.

Le système mesure la fréquence de résonance Fr pour chacun des modes de vibration de l'oscillateur. Comme indiqué ci-dessus, toute espèce cible qui se greffe sur une bille B_1, B_2, B_3 piégée dans l'oscillateur 11, entraîne l'augmentation de la masse de celui-ci, ce qui diminue sa fréquence de résonance, d'un décalage proportionnel à la masse de la cible. En mesurant les fluctuations de fréquence de résonance en continu, il est alors possible de remonter à la masse adsorbée sur l'oscillateur en temps réel et suivre ainsi des cinétiques d'adsorption avec des espèces cibles. Pour chacun des modes de vibration M_1, M_2, M_3 de l'oscillateur 11, on constate en effet une variation de la fréquence de résonance ΔFr_1, ΔFr_2, ΔFr_3.

La deuxième configuration de fonctionnement est décrite ci-dessous en liaison avec la figure 4.

Pour cette autre configuration, on utilise des billes B_1, B_2 de tailles différentes, et disposant d'une surface fonctionnalisée par des sondes différentes. En effet, les billes sont fonctionnalisées en surface pour présenter des sondes complémentaires de cibles dont on souhaite détecter la présence dans le milieu circulant dans le circuit microfluidique 10. En utilisant des billes de tailles et sonde différentes, on pourra adresser la capacité de mesurer l'adsorption de plusieurs cibles différentes.

L'oscillateur 11 comporte par exemple deux pièges fluidiques de dimensions distinctes.

E10 : On injecte d'abord les plus petites billes, c'est-à-dire celles qui sont dimensionnées de sorte qu'elles ne se logent que dans les plus petites restrictions et passent à travers les plus grosses restrictions pour ne pas obstruer ces dernières. Ensuite on injecte les billes les plus grosses. L'obstruction des pièges occupés préalablement par les plus petites billes va empêcher d'y loger les plus grosses. On pourra réaliser cette séquence N fois, N étant le nombre de types de billes possibles (de diamètre et nature de sonde donné) auquel on associe des restrictions de largeur adaptée. A titre d'exemple, on vient placer deux billes distinctes dans deux pièges fluidiques distincts de l'oscillateur. La bille B_1 la plus petite vient ainsi se loger dans le piège ayant la restriction la plus étroite et la bille B2 plus grosse vient se loger dans le piège ayant la plus restriction la plus large.

E20 : On excite l'oscillateur 11 selon les différents modes de vibration M_1, M_2 pour enregistrer la fréquence de résonance Fr de l'oscillateur.

En confinant les différentes billes B_1, B_2 à différentes positions dans le capteur, sur des anti-nœuds pour amplifier le signal de masse, on peut par approche multimodale déduire la variation de masse absorbée sur chaque bille et ainsi remonter à la concentration en espèces cibles qui se sera absorbée sur la bille correspondante.

En confinant les billes sur les différents pièges fluidiques de l'oscillateur, c'est-à-dire sur les anti-nœuds liés à chaque mode de vibration, il est possible de déduire la variation de masse absorbée sur chaque particule et ainsi de remonter à la concentration en espèces cibles absorbée sur le piège fluidique adressé.

Dans l'exemple repris sur la figure 4, une première bille B_1 se place ainsi sur un anti-nœud lié au premier mode de vibration M_1 de l'oscillateur. La seconde bille B_2 se place sur un anti-nœud lié au deuxième mode de vibration M_2 de l'oscillateur, cette position étant dans cet exemple proche d'un nœud de vibration pour le premier mode de vibration M_1 ce qui produira peu de signal sur ce mode de vibration résultant de l'adsorption de masse.

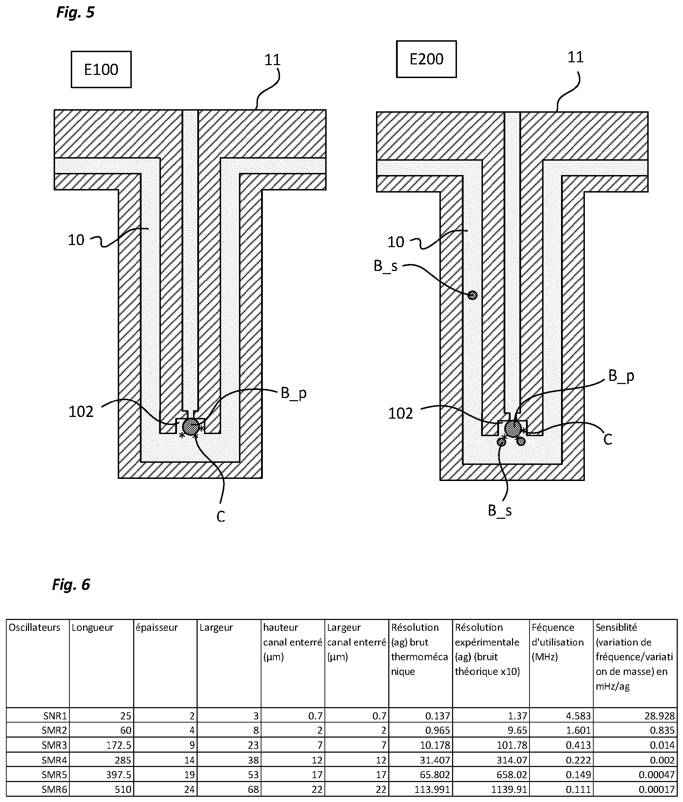

Selon une autre configuration de fonctionnement illustrée par la figure 5, pour renforcer le signal (amplification par la masse et accroître la spécificité), on pourra également utiliser une deuxième famille de billes recouvertes d'anticorps secondaires, complémentaires des cibles C préalablement accrochées aux billes B_p déjà piégées dans l'oscillateur. De cette manière on dispose de billes dites primaires B_p, initialement piégées dans l'oscillateur (figure 5 - E100), et de billes dites secondaires B_s portant des anticorps secondaires. Ces billes secondaires B_s viennent ainsi se greffer sur les billes primaires B_p (figure 5 - E200).

Le gradient de masse, consécutif à l'adsorption avec une bille secondaire B_s sur chaque cible C greffée sur la ou les billes primaires B_p, sera significativement plus important qu'avec un anticorps secondaire seul greffé sur les billes primaires B_p.

Par exemple, on prend un design (noté SMR6 dans le tableau de la figure 6) ayant une résolution expérimentale de l'ordre 1,139fg (1 femtogramme =10^-15g), compatible avec la pesée d'une bille secondaire d'or de 60nm de diamètre dont la masse flottante dans l'eau est de 2,07fg (pour une bille d'or de 70nm, sa masse flottante est de 3,287fg). En comparaison, les anticorps classiques ont typiquement une masse autour de quelques dizaines de kiloDalton (par ex. 100kDa=0,166ag), et donc un seul anticorps est indétectable avec l'oscillateur SMR6. Dans le cas d'un oscillateur ayant la configuration notée SMR2, sa résolution expérimentale est estimée autour de 9,65 ag, ce qui est compatible avec la pesée d'une bille secondaire d'or de 15nm (masse flottante 32ag) ou encore 20nm (masse flottante 76ag).

Le principe de fonctionnement de cette configuration est illustré par la figure 5 avec un oscillateur disposant d'un seul piège fluidique 102.

On pourra appliquer ce principe aux variantes listées ci-dessus (c'est-à-dire avec le piégeage de plusieurs billes "primaires" de tailles distinctes ou non) pour renforcer le signal relatif à l'adsorption des cibles (distinctes ou non) au sein d'un seul et même capteur. On pourra utiliser des billes secondaires de masses potentiellement identiques/proches (ou de distribution en masse qui se chevauche) pour chacune des cibles correspondantes, dans la mesure où la détection multimodale permet de connaître précisément l'emplacement de la bille primaire où chacune de ses billes secondaires sont greffées.

Le tableau de la figure 6 résume les performances attendues (résolution en masse) pour des capteurs référencés SNR1 à SMR6, en tenant compte uniquement du bruit thermomécanique, d'une amplitude d'oscillation à la limite de non-linéarité mécanique et en supposant un facteur de qualité conservateur Q=1000 avec une fréquence d'échantillonnage du mHz pour les mesures de fluctuations en temps réel.

En multipliant par un facteur 10 conservateur la résolution théorique limitée par le bruit thermomécanique, on peut raisonnablement s'attendre à ce que la résolution expérimentale des capteurs soit plutôt autour de 1ag à 1fg (pour les designs respectifs SNR1 et SMR6). C'est ce seuil que l'on souhaitera atteindre pour être en mesure de détecter des biomarqueurs par adsorption sur la bille et quantifier un temps de réponse attendu.

L'invention présente ainsi de nombreux avantages. On peut ainsi piéger plusieurs particules au sein d'un même oscillateur, ces particules pouvant par exemple se distinguer les unes des autres par leur diamètre et/ou par la fonctionnalisation de leurs surfaces respectives. Cette approche multi-particules et donc multi-sondes permettra de réaliser une détection multi-cibles au sein d'un seul et même oscillateur.

L'invention est par ailleurs adaptable à différentes configurations d'oscillateur.

## Revendications

1. Procédé de détection d'une première cible et d'une deuxième cible, mis en œuvre à l'aide d'un système de détection qui comporte :

   - Un dispositif de mesure (1_A, 1_B) qui comporte un oscillateur (11_A, 11_B) mécanique et un circuit fluidique (10_A, 10B) intégré à l'oscillateur et dans lequel est amené à circuler un fluide contenant ladite première cible et ladite deuxième cible à détecter, ledit oscillateur (11_A, 11_B) étant apte à être excité selon plusieurs modes de vibration (M_1, M_2, M_3) et comportant sur le réseau fluidique au moins un premier piège positionné sur un anti-nœud d'un premier mode de vibration de l'oscillateur et un deuxième piège positionné sur un anti-nœud d'un deuxième mode de vibration de l'oscillateur, un anti-nœud de vibration correspondant à une position dans laquelle l'amplitude de vibration va de 80% à 100% de l'amplitude maximale pour le mode de vibration sélectionné,
   - Des moyens d'injection d'un fluide dans le circuit fluidique,
   - Des moyens d'excitation (M_Exc) dudit oscillateur selon le premier mode de vibration et/ou le deuxième mode de vibration,
   - Des moyens de mesure (M_Fr) de la fréquence de résonance de l'oscillateur.

   Ledit procédé comportant des étapes de :

- Piégeage d'une première particule (B_1) dans le premier piège de l'oscillateur et piégeage d'une deuxième particule (B_2) dans le deuxième piège de l'oscillateur, ladite première particule (B_1) disposant d'une surface externe fonctionnalisée à l'aide d'une première sonde complémentaire de ladite première cible et ladite deuxième particule (B_2) disposant d'une surface externe fonctionnalisée à l'aide d'une deuxième sonde complémentaire de ladite deuxième cible,

- Injection du fluide dans le circuit fluidique, ledit fluide contenant ladite première cible et ladite deuxième cible à détecter,

- Première excitation de l'oscillateur selon le premier mode de vibration (M_1) et mesure d'une première fréquence de résonance de l'oscillateur,

- Détection de ladite première cible à partir d'une variation (ΔFr_1) de ladite première fréquence de résonance,

- Deuxième excitation de l'oscillateur selon le deuxième mode de vibration (M_2) et mesure d'une deuxième fréquence de résonance de l'oscillateur,

- Détection de ladite deuxième cible à partir d'une variation (ΔFr_2) de ladite deuxième fréquence de résonance.

2. Procédé selon la revendication 1, **caractérisé en ce que** la première sonde et la deuxième sonde sont distinctes pour capturer respectivement la première cible et la deuxième cible qui sont distinctes.

3. Procédé selon la revendication 1, **caractérisé en ce que** la première sonde cible et la deuxième sonde sont identiques pour capturer la première cible et la deuxième cible qui sont identiques.

4. Procédé selon la revendication 2 ou 3, **caractérisé en ce qu'**il comporte une étape d'injection dans le circuit fluidique de particules dites secondaires fonctionnalisées chacune par une sonde spécifique de la première cible et/ou de la deuxième cible.

5. Procédé selon l'une des revendications 1 à 4, **caractérisé en ce que** chaque particule est réalisée sous la forme d'une bille rigide.

6. Procédé selon la revendication 5, **caractérisé en ce que** les billes sont de tailles différentes.

7. Procédé selon l'une des revendications 1 à 6, **caractérisé en ce que** la première excitation et la deuxième excitation sont réalisées de manière simultanée.

8. Système de détection employé pour mettre en œuvre le procédé de détection de cibles tel que défini dans l'une des revendications précédentes, ledit système comprenant :

- Un dispositif de mesure (1_A, 1_B) qui comporte un oscillateur (11_A, 11_B) mécanique et un circuit fluidique (10_A, 10_B) intégré à l'oscillateur et dans lequel est amené à circuler un fluide contenant les particules à caractériser, ledit oscillateur étant apte à être excité selon plusieurs modes de vibration (M_1, M_2) et comportant sur le réseau fluidique au moins un premier piège positionné sur un anti-nœud d'un premier mode de vibration de l'oscillateur et un deuxième piège positionné sur un anti-nœud d'un deuxième mode de vibration de l'oscillateur, un anti-nœud de vibration correspondant à une position dans laquelle l'amplitude de vibration va de 80% à 100% de l'amplitude maximale pour le mode de vibration sélectionné,

- Des moyens d'injection d'un fluide dans le circuit fluidique,

- Des moyens d'excitation (M_Exc) commandés de manière à pouvoir exciter ledit oscillateur selon le premier mode de vibration et/ou le deuxième mode de vibration pour la mise en œuvre du procédé tel que défini dans l'une des revendications précédentes,

- Des moyens de mesure (M_Fr) de la fréquence de résonance de l'oscillateur.

9. Système selon la revendication 8, **caractérisé en ce qu'**il comporte des moyens de détection de la variation de la fréquence de résonance.

10. Système selon la revendication 8 ou 9, **caractérisé en ce que** l'oscillateur (11_A, 11_B) comporte une poutre fixée en porte à faux et qui intègre le circuit fluidique (10_A, 10_B).

11. Système selon la revendication 10, **caractérisé en ce que** le circuit fluidique (10_A) comporte un canal principal (100_A) et un canal intermédiaire (101_A), chaque piège fluidique (102_A) étant réalisé sous la forme d'une cavité entre le canal principal et le canal intermédiaire, qui comporte une partie élargie (103_A) prolongée d'une restriction (104_A).

12. Système selon la revendication 10, **caractérisé en ce que** le circuit fluidique comporte un canal principal s'étendant entre une entrée fluidique et une sortie fluidique, le canal principal comportant une première branche (100_B) dans laquelle débouche l'entrée fluidique, une deuxième branche (101_B) prolongeant ladite première branche, via une branche de jonction (105_B), et débouchant sur la sortie fluidique, chaque piège fluidique (102_B) étant réalisé sous la forme d'une branche de dérivation par rapport au canal principal et comportant un logement (103_B) dimensionné pour accueillir une particule et une restriction (104_B) prolongeant ledit logement.

**Patentansprüche**

1. Verfahren zur Detektion eines ersten Targets und eines zweiten Targets, das mithilfe eines Detektionssystems durchgeführt wird, das umfasst:

   - Eine Messvorrichtung (1_A, 1_B), die einen mechanischen Oszillator (11_A, 11_B) umfasst und einen Fluidkreislauf (10_A, 10B), der in den Oszillator integriert ist und in dem ein Fluid zirkulieren soll, welches das erste Target und das zweite Target, die zu detektieren sind, enthält, wobei der Oszillator (11_A, 11_B) geeignet ist, gemäß mehreren Schwingungsmoden (M_1, M_2, M_3) angeregt zu werden, und an dem Fluidnetz mindestens eine an einem Bauch einer ersten Schwingungsmode des Oszillators positionierte erste Falle und eine an einem Bauch einer zweiten Schwingungsmode des Oszillators positionierte zweite Falle umfasst, wobei ein Schwingungsbauch einer Position entspricht, an der die Schwingungsamplitude 80 % bis 100 % der maximalen Amplitude für die ausgewählte Schwingungsmode beträgt,
   - Mittel zum Einleiten eines Fluids in den Fluidkreislauf,
   - Mittel zum Anregen (M_Exc) des Oszillators gemäß der ersten Schwingungsmode und/oder der zweiten Schwingungsmode,
   - Mittel zum Messen (M_Fr) der Resonanzfrequenz des Oszillators.

   Wobei das Verfahren die folgenden Schritte umfasst:

   - Einfangen eines ersten Teilchens (B_1) in der ersten Falle des Oszillators und Einfangen eines zweiten Teilchens (B_2) in der zweiten Falle des Oszillators, wobei das erste Teilchen (B_1) über eine Außenfläche verfügt, die mithilfe einer zu dem ersten Target komplementären ersten Sonde funktionalisiert ist, und wobei das zweite Teilchen (B_2) über eine Außenfläche verfügt, die mithilfe einer zu dem zweiten Target komplementären zweiten Sonde funktionalisiert ist,
   - Einleiten des Fluids in den Fluidkreislauf, wobei das Fluid das erste Target und das zweite Target, die zu detektieren sind, enthält,
   - Erstes Anregen des Oszillators gemäß der ersten Schwingungsmode (M_1) und Messen einer ersten Resonanzfrequenz des Oszillators,
   - Detektieren des ersten Targets anhand einer Änderung (ΔFr_1) der ersten Resonanzfrequenz,

   - Zweites Anregen des Oszillators gemäß der zweiten Schwingungsmode (M_2) und Messen einer zweiten Resonanzfrequenz des Oszillators,
   - Detektieren des zweiten Targets anhand einer Änderung (ΔFr_2) der zweiten Resonanzfrequenz.

2. Verfahren nach Anspruch 1, **dadurch gekennzeichnet, dass** die erste Sonde und die zweite Sonde verschieden sind, um das erste Target beziehungsweise das zweite Target, die verschieden sind, zu erfassen.

3. Verfahren nach Anspruch 1, **dadurch gekennzeichnet, dass** die erste Targetsonde und die zweite Sonde identisch sind, um das erste Target und das zweite Target, die identisch sind, zu erfassen.

4. Verfahren nach Anspruch 2 oder 3, **dadurch gekennzeichnet, dass** es einen Schritt des Einleitens in den Fluidkreislauf von sogenannten sekundären Teilchen umfasst, von denen jedes durch eine spezifische Sonde des ersten Targets und/oder des zweiten Targets funktionalisiert ist.

5. Verfahren nach einem der Ansprüche 1 bis 4, **dadurch gekennzeichnet, dass** jedes Teilchen in Form einer starren Kugel ausgeführt ist.

6. Verfahren nach Anspruch 5, **dadurch gekennzeichnet, dass** die Kugeln unterschiedlich groß sind.

7. Verfahren nach einem der Ansprüche 1 bis 6, **dadurch gekennzeichnet, dass** das erste Anregen und das zweite Anregen gleichzeitig ausgeführt werden.

8. Detektionssystem, das dazu eingesetzt wird, das in einem der vorhergehenden Ansprüche definierte Verfahren zur Detektion von Targets durchzuführen, das System umfassend:

   - Eine Messvorrichtung (1_A, 1_B), die einen mechanischen Oszillator (11_A, 11_B) umfasst und einen Fluidkreislauf (10_A, 10_B), der in den Oszillator integriert ist und in dem ein Fluid zirkulieren soll, welches die zu charakterisierenden Teilchen enthält, wobei der Oszillator geeignet ist, gemäß mehreren Schwingungsmoden (M_1, M_2) angeregt zu werden, und an dem Fluidnetz mindestens eine an einem Bauch einer ersten Schwingungsmode des Oszillators positionierte erste Falle und eine an einem Bauch einer zweiten Schwingungsmode des Oszillators positionierte zweite Falle umfasst, wobei ein Schwingungsbauch einer Position entspricht, an der die Schwingungsamplitude 80 % bis 100 % der maximalen Amplitude für die ausgewählte Schwingungsmode beträgt,
- Mittel zum Einleiten eines Fluids in den Fluidkreislauf,
- Mittel zum Anregen (M_Exc), die so gesteuert werden, dass sie den Oszillator zur Durchführung des in einem der vorhergehenden Ansprüche definierten Verfahrens gemäß der ersten Schwingungsmode und/oder der zweiten Schwingungsmode anregen können,
- Mittel zum Messen (M_Fr) der Resonanzfrequenz des Oszillators.

9. System nach Anspruch 8 **dadurch gekennzeichnet, dass** es Mittel zum Detektieren der Änderung der Resonanzfrequenz umfasst.

10. System nach Anspruch 8 oder 9, **dadurch gekennzeichnet, dass** der Oszillator (11_A, 11_B) einen Balken umfasst, der vorkragend befestigt ist und der den Fluidkreislauf (10_A, 10_B) beinhaltet.

11. System nach Anspruch 10, **dadurch gekennzeichnet, dass** der Fluidkreislauf (10_A) einen Hauptkanal (100_A) und einen Zwischenkanal (101_A) umfasst, wobei jede Fluidfalle (102_A) in Form eines Hohlraums zwischen dem Hauptkanal und dem Zwischenkanal ausgeführt ist, der einen aufgeweiteten Teil (103_A) umfasst, der mit einer Verengung (104_A) verlängert wird.

12. System nach Anspruch 10, **dadurch gekennzeichnet, dass** der Fluidkreislauf einen Hauptkanal umfasst, der sich zwischen einem Fluideinlass und einem Fluidauslass erstreckt, wobei der Hauptkanal einen ersten Zweig (100_B) umfasst, in den der Fluideinlass mündet, wobei ein zweiter Zweig (101_B) den ersten Zweig über einen Übergangszweig (105_B) verlängert und in den Fluidauslass mündet, wobei jede Fluidfalle (102_B) in Form eines Bypasszweigs in Bezug auf den Hauptkanal ausgeführt ist und eine zum Aufnehmen eines Teilchens dimensionierte Aufnahme (103_B) und eine die Aufnahme verlängernde Verengung (104_B) umfasst.

**Claims**

1. Method for detecting a first target and a second target, implemented using a detection system that comprises:

   - a measuring device (1_A, 1_B), which comprises a mechanical oscillator (11_A, 11_B) and a fluidic circuit (10_A, 10B) incorporated into the oscillator and in which a fluid containing said first target and said second target to be detected is circulated, said oscillator (11_A, 11_B) being capable of being excited according to a plurality of vibration modes (M_1, M_2, M_3) and comprising, in the fluidic network, at least a first trap positioned at an antinode of a first vibration mode of the oscillator and a second trap positioned at an antinode of a second vibration mode of the oscillator, a vibration antinode corresponding to a position at which the vibration amplitude ranges from 80% to 100% of the maximum amplitude for the selected vibration mode,
   - means for injecting a fluid into the fluidic circuit,
   - excitation means (M_Exc) for exciting said oscillator according to the first vibration mode and/or the second vibration mode,
   - measuring means (M_Fr) for measuring the resonant frequency of the oscillator,

   said method comprising the steps of:

   - trapping a first particle (B_1) in the first trap of the oscillator and trapping a second particle (B_2) in the second trap of the oscillator, said first particle (B_1) having an external surface that is functionalized using a first probe that is complementary to said first target, and said second particle (B_2) having an external surface that is functionalized using a second probe that is complementary to said second target,
   - injecting the fluid into the fluidic circuit, said fluid containing said first target and said second target to be detected,
   - first excitation of the oscillator according to the first vibration mode (M_1) and measuring a first resonant frequency of the oscillator,
   - detecting said first target on the basis of a variation ($\Delta Fr\_1$) in said first resonant frequency,
   - second excitation of the oscillator according to the second vibration mode (M_2) and measuring a second resonant frequency of the oscillator,
   - detecting said second target on the basis of a variation ($\Delta Fr\_2$) in said second resonant frequency.

2. Method according to Claim 1, **characterized in that** the first probe and the second probe are distinct in order to respectively capture the first target and the second target, which are distinct.

3. Method according to Claim 1, **characterized in that** the first target probe and the second probe are identical in order to capture the first target and the second target, which are identical.

4. Method according to Claim 2 or 3, **characterized in**

**that** it comprises a step of injecting, into the fluidic circuit, particles referred to as secondary particles, which are each functionalized by a probe specific to the first target and/or the second target.

5. Method according to one of Claims 1 to 4, **characterized in that** each particle takes the form of a rigid bead.

6. Method according to Claim 5, **characterized in that** the beads have different sizes.

7. Method according to one of Claims 1 to 6, **characterized in that** the first excitation and the second excitation are carried out simultaneously.

8. Detection system employed for implementing the target detection method as defined in one of the preceding claims, said system comprising:

   - a measuring device (1_A, 1_B), which comprises a mechanical oscillator (11_A, 11_B) and a fluidic circuit (10_A, 10_B) incorporated into the oscillator and in which a fluid containing the particles to be characterized is circulated, said oscillator being capable of being excited according to a plurality of vibration modes (M_1, M_2) and comprising, in the fluidic network, at least a first trap positioned at an antinode of a first vibration mode of the oscillator and a second trap positioned at an antinode of a second vibration mode of the oscillator, a vibration antinode corresponding to a position at which the vibration amplitude ranges from 80% to 100% of the maximum amplitude for the selected vibration mode,
   - means for injecting a fluid into the fluidic circuit,
   - excitation means (M_Exc), which are controlled so as to be able to excite said oscillator according to the first vibration mode and/or the second vibration mode in order to implement the method as defined in one of the preceding claims,
   - measuring means (M_Fr) for measuring the resonant frequency of the oscillator.

9. System according to Claim 8, **characterized in that** it comprises means for detecting the variation in the resonant frequency.

10. System according to Claim 8 or 9, **characterized in that** the oscillator (11_A, 11_B) comprises a cantilevered beam that incorporates the fluidic circuit (10_A, 10_B).

11. System according to Claim 10, **characterized in that** the fluidic circuit (10_A) comprises a main channel (100_A) and an intermediate channel (101_A), each fluidic trap (102_A) taking the form of a cavity that is between the main channel and the intermediate channel and comprises an enlarged portion (103_A) extended by a constriction (104_A).

12. System according to Claim 10, **characterized in that** the fluidic circuit comprises a main channel extending between a fluidic inlet and a fluidic outlet, the main channel comprising a first branch (100_B) into which the fluidic inlet opens out, a second branch (101_B) extending said first branch, via a junction branch (105_B), and opening out at the fluidic outlet, each fluidic trap (102_B) taking the form of a branch that bypasses the main channel and comprises a housing (103_B) dimensioned to receive a particle, and a constriction (104_B) extending said housing.

**Fig. 1A**

**Fig. 1B**

**Fig. 2**

*Fig. 3*

E1

11

L →

10

B_1

102

B_2

B_3

E2

11

L →  →

102

M_1

M_2

M_3

A_1   A_2   A_3

— — — — M_1

———— M_2

— · — · M_3

M_1    Fr      ΔFr_1                t

M_2    Fr      ΔFr_2                t

M3     Fr      ΔFr_3                t

**Fig. 4**

E10

E20

*Fig. 5*

*Fig. 6*

| Oscillateurs | Longueur | épaisseur | Largeur | hauteur canal enterré (µm) | Largeur canal enterré (µm) | Résolution (ag) brut thermoméca nique | Résolution expérimentale (ag) (bruit théorique x10) | Féquence d'utilisation (MHz) | Sensiblité (variation de fréquence/variati on de masse) en mHz/ag |
|---|---|---|---|---|---|---|---|---|---|
| SNR1 | 25 | 2 | 3 | 0.7 | 0.7 | 0.137 | 1.37 | 4.583 | 28.928 |
| SMR2 | 60 | 4 | 8 | 2 | 2 | 0.965 | 9.65 | 1.601 | 0.835 |
| SMR3 | 172.5 | 9 | 23 | 7 | 7 | 10.178 | 101.78 | 0.413 | 0.014 |
| SMR4 | 285 | 14 | 38 | 12 | 12 | 31.407 | 314.07 | 0.222 | 0.002 |
| SMR5 | 397.5 | 19 | 53 | 17 | 17 | 65.802 | 658.02 | 0.149 | 0.00047 |
| SMR6 | 510 | 24 | 68 | 22 | 22 | 113.991 | 1139.91 | 0.111 | 0.00017 |

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2574885 A1

**Littérature non-brevet citée dans la description**

- **Y.C. WENG** ; **F.F. DELGADO** ; **S. SON** ; **T.P. BURG** ; **S.C. WASSERMAN** ; **S.R. MANALIS**. Mass sensors with mechanical traps for weighing single cells in different fluids. *Lab on a chip*, 2011